# FASCICULE DE BREVET EUROPEEN

(11) **EP 1 221 929 B1**
(45) Date de publication et mention de la délivrance du brevet: **06.09.2006**
(21) Numéro de dépôt: 00966258.6
(22) Date de dépôt: 03.10.2000
(51) Int. Cl.: A61Q 1/02, A61Q 1/04, A61Q 1/06, A61K 8/19, A61K 8/25, A61K 8/26, A61K 8/24, A61K 8/81, A61K 8/85, A61K 8/86, A61K 8/87, A61K 8/88, A61K 8/89, A61K 8/73, A61K 8/91, A61K 8/28, A61K 8/29

(54) **PROCEDE D'AMELIORATION DES PROPRIETES DE NON-TRANSFERT DE COMPOSITIONS COSMETIQUES**
VERFAHREN ZUR VERBESSERUNG DER ABRIEBFESTIGKEIT VON KOSMETISCHEN ZUSAMMENSETZUNGEN
METHOD FOR IMPROVING THE STAY-ON PROPERTIES OF COSMETIC COMPOSITIONS

(30) Priorité: 21.10.1999 FR 9913140
(43) Date de publication de la demande: 17.07.2002
(73) Titulaire: L'ORÉAL, 75008 Paris (FR)
(72) Inventeur: QUINN, Francis, Xavier, F-75005 Paris (FR); GIUSTINIANI, Pascal, F-92300 Levallois-Perret (FR); JEANNE ROSE, Valérie, F-75019 Paris (FR)
(74) Mandataire: Casalonga, Axel
(86) Numéro de dépôt international: PCT/FR2000/002732
(87) Numéro de publication internationale: WO 2001/028504

(56) Documents cités:
- EP-A- 0 117 360
- EP-A- 0 429 325
- EP-A- 0 669 362
- WO-A-98/44906

## Description

La présente invention concerne un procédé d'amélioration des propriétés de non-transfert de compositions cosmétiques susceptibles d'être appliquées sur la peau, ou les lèvres utilisant un matériau hybride organo-minéral.

Dans le domaine cosmétique et en particulier dans le domaine du maquillage, il existe depuis de nombreuses années des tentatives pour améliorer la tenue des compositions appliquées sur la peau et/ou les lèvres. Les formules dites "non-transfert" mises au point présentent des inconvénients majeurs qui sont leur faible brillance propriété particulièrement indésirable dans le domaine des rouges a lèvres, ainsi qu'un certain inconfort d'utilisation ressenti en particulier pour les fonds de teints. La plupart de ces produits sont en outre difficiles à éliminer par des produits de démaquillage habituels.

La demanderesse a découvert qu'il était possible d'améliorer les propriétés de non-transfert de compositions cosmétiques, notamment de soin ou de maquillage, en leur associant certains matériaux hybrides organo-minéraux, élaborés par voie sol-gel.

Ces matériaux hybrides, lorsqu'ils sont incorporés dans ou appliqués sur des compositions cosmétiques existantes, améliorent significativement la tenue de ces compositions, empêchent tout transfert parcontact de la peau ou des lèvres vers un substrat (cigarette, vaisselle, vêtements etc.), peuvent augmenter la brillance de ces compositions et permettent un grand confort d'utilisation. Le maquillage s'élimine par ailleurs facilement avec un démaquillant usuel.

La présente invention a par conséquent pour objet un procédé pour améliorer les propriétés de non-transfert d'une composition cosmétique appliquée sur la peau et les lèvres caractérisé par le fait que que l'on recouvre ladite composition cosmétique par un matériau hybride organo-minéral obtenu par voie sol-gel à partir d'un mélange comprenant :
(A) au moins un composé métallique ou métallo-organique, et
(B) au moins polymère organique ou siliconé choisi parmi un homopolymère ou un copolymère statistique séquencés et/ou greffé choisi parmi :
   (a) les homopolymères et copolymères d'alkyloxazoline ;
   (b) les homopolymères et copolymères d'acide (méth)acrylique, d'acide crotonique d'acide maléique d'acide itaconique d'acide styrène-sulfonique d'acide le acrylamido-2-méthylpropane-sulfonique, de méthacrylate de 2-sulfoéthyle, d'acide vinylsulfonique et/ou d'acide vinylphosphonique ;
   (c) les homopolymères d'esters ou d'amides acryliques ou méthacryliques et leurs copolymères avec des comonomères choisis parmi les acides carboxyliques insaturés, les acides sulfoniques, les acides phosphoniques, les esters et éthers vinyliques, les oléfines, le styrène les styrènes substitués les fluoro- ou perfluorooléfines, les méth)acrylates de perfluoroalkyle, les composés vinyliques fluorés et les organosilanes, organosiloxanes ou organopolysiloxanes insaturés ;
   (d) les homopolymères et copolymères d'alcool vinylique,
   (e) les homopolymères d'esters ou d'amides vinyliques et/ou allyliques et/ou méthallyliques et leurs copolymères avec des comonomères choisis parmi les acides carboxyliques insaturés les acides sulfoniques, les acides phosphoniques, les esters et éthers vinyliques, les oléfines, les styrène, les styrènes substitués les fluoro- et perfluorooléfines, les (méth)acrylates de perfluoroalkyle, les composés vinyliques fluorés et les organosilanes, organosiloxanes ou organopolysiloxanes insaturés :
   (f) les polyéthers ;
   (g) les polyesters ;
   (h) les homo- et copolymères d'oléfines ou de cyclooléfines ;
   (i) les polyamides et polyesteramides ;
   (j) les polyuréthannes et les polyurées pouvant comporter des séquences polyéther, polyester et/ou polyorganosiloxane ;
   (k) les polymères fluorés ;
   (l) les polymères naturels et les polymères naturels modifiés (polymères artificiels) ;
   (m) les polyorganosiloxanes ;
   (n) les polyorganophosphazènes ;
   (o) les polysilanes -(SiR^{a}R^{b}-)ₙ, les polycarbosilanes -(SiR^{a}R^{b-}CR^{c}R^{d}-)ₙ et les polysilazanes -(-SiR^{a}R^{b}-NR^{c})ₙ, et,
   (p) des mélanges de ces polymères,
   ou un précurseur de ceux-ci
formant après évaporation de la fraction volatile une structure réticulée.

Le matériau hybride organo-minéral utilisé dans la présente invention pour améliorer les propriétés de non-transfert de compositions cosmétiques, notamment de soin ou de maquillage, est connu et a été décri dans la demande de brevet français n° 97 04157 et dans la EPO 117360. Il s'agit d'un matériau formé par des nanoparticules minérales reliées entre elles par des chaînes de polymères organiques ou siliconés.

Ce matériau est obtenu selon un procédé dit "sol-gel" c'est-à-dire un procédé impliquant l'hydrolyse partielle ou complète, puis la condensation des ingrédients d'une solution de départ, la solution de départ donnant, après hydrolyse et condensation, le matériau hybride utilisé dans le procédé de la présente invention :

Dans la présente invention, le mélange des composants (A) et (B) donnant, après hydrolyse et condensation, ledit matériau hybride comprend de préférence en outre au moins un solvant (composant (C)).

Le composant (A), lorsqu'il s'agit d'un composé métallique, peut être choisi parmi
(i) les oxydes des métaux de transition des groupes IB à VIIB ou du groupe des lanthanides de la classification périodique des éléments,
(ii) les oxydes d'aluminium, de bore, de silicium ou d'étain, et
(iii) les phosphates d'aluminium.

Des composés métalliques préférés utilisables dans la présente invention sont choisis dans le groupe (i) formé par les oxydes des métaux de transition et englobent en particulier l'oxyde de titane, l'oxyde de fer et l'oxyde de zirconium.

Ce composant (A) minéral doit être réactif vis-à-vis du composant (B) et permettre la formation de liaisons covalentes et/ou physiques de manière à former un réseau tridimensionnel.

Lorsqu'il s'agit d'un composé métallo-organique, le composant (A) peut être choisi dans les trois groupes de composés suivants :
(1) les composés correspondant à l'une des formules suivantes :

   (Ia) M(OR₁)ₙ

   (Ib) R-M-(OR₁)ₙ₋₁

   (Ic) (OR₁)ₙ₋₁-M-R"-M(OR₁)ₙ₋₁

   et 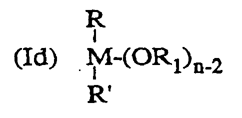 où
   M représente un atome d'un métal de transition des groupes IB à VIIB de la classification périodique des éléments ou un atome d'aluminium, de bore, de silicium ou d'étain, de préférence un atome de Si, Ti ou Zr,
   n est égal à la valence de M,
   R₁ représente un radical alkyle linéaire ou ramifié en C₁₋₃₀, de préférence en C₁₋₆,
   R et R' représentent, indépendamment l'un de l'autre, un groupe alkyle linéaire ou ramifié, un groupe cycloalkyle ou un groupe aryle substitué ou non, lesdits groupes R et R' pouvant eux-mêmes être capables de réagir ou pouvant porter un substituant susceptible de réagir avec le polymère organique ou siliconé fonctionnalise (B), et lesdits groupes R et R' pouvant porter en plus un groupe cosmétiquement ou dermatologiquement actif choisi, par exemple, parmi un groupe colorant, un groupe photochromique, un groupe filtrant le rayonnement UV, un groupe favorisant l'adhérence sur les matières kératiniques (tels que des groupes de type amide, uréthane, urée, hydroxy, carboxy, acide aminé ou polypeptide), un groupe facilitant le démaquillage, un groupe bactéricide, un groupe chélatant pouvant en particulier complexer des cations multivalents, un hydroxyacide, un neuro-suppresseur, un groupe anti-chute des cheveux, un groupe antioxydant, un groupe anti-radicaux libres ou un groupe porteur de vitamine,
   R" représente un radical alkylène linéaire ou ramifié, cycloalkylène ou arylène éventuellement substitué, pouvant réagir lui-même ou pouvant porter un substituant capable de réagir avec le polymère organique ou siliconé fonctionnalisé (B), tel que ceux définis ci-après, et pouvant porter en plus un groupe cosmétiquement ou dermatologiquement actif tel que ceux définis ci-dessus pour R et R';
(2) les complexes de coordination des composés de formule (Ia) à (Id) ci-dessus, correspondant à une des formules suivantes

   (IIa) M(OR₁)ₙ₋ₓ (X)ₓ

   (IIb) R-M(OR₁)ₙ₋₁₋ₓ (X)ₓ

   (IIC) (X)ₓ (OR₁)ₙ₋₁₋ₓM-R"-M-(OR₁)ₙ₋₁₋ₓ (X)ₓ

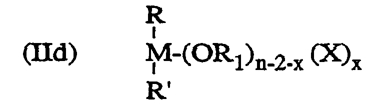

   où
   M représente un atome d'un métal de transition des groupes IB à VIIB de la classification périodique des éléments ou un atome d'aluminium, de bore, de silicium ou d'étain, de préférence un atome de Ti ou Zr,
   X est un ligand monocoordiné ou monodenté comportant un atome d'azote, un atome de phosphore, un atome de soufre ou un atome d'oxygène, et pouvant porter un substituant susceptible de réagir avec ledit polymère organique fonctionnalisé ou ledit polymère silicone fonctionnalisé (B), tel que ceux définis ci-dessus.
   x représente le nombre de ligands X, et
   n, R, R₁, R' et R" sont tels que définis pour les formules (Ia) à (Id), et
(3) les complexes de chélation répondant à la formule suivante :

   (III) M(OR₁)_{n-bx} (X')ₓ

   dans laquelle
   M représente un atome d'un métal de transition des groupes IB à VIIB de la classification périodique des éléments ou un atome d'aluminium, de bore, de silicium ou d'étain, de préférence un atome de Ti ou Zr,
   X' représente un groupe chélatant ou ligand polydenté comportant un atome d'azote, un atome de phosphore, un atome de soufre et/ou un atome d'oxygène, et pouvant porter un substituant susceptible de réagir avec ledit polymère organique fonctionnalisé ou ledit polymère siliconé fonctionnalisé (B), tel que ceux définis ci-après, et pouvant porter un groupe cosmétiquement ou dermatologiquement actif tel que ceux définis ci-dessus pour R et R',
   b correspond à la valence de coordination du ligand X' et est au moins égal à 2,
   x est égal au nombre de ligands X', et
   R₁ et n ont les significations indiquées ci-dessus pour les formules (Ia) à (Id).

Dans la description des complexes de composés métallo-organiques ci-dessus, on entend par ligand monocoordiné ou monodenté (X) un groupe comportant un seul atome pouvant se lier à l'atome central métallique. On peut citer à titre d'exemples de tels ligands, les acides carboxyliques et les cétones.

Par groupe chélatant (X') selon la présente invention, on entend un ligand polydenté lié à un seul atome métallique central par plus d'un atome donneur de doublet d'électrons. Il est choisi de préférence parmi les β-dicétones, les β-cétoesters, les β-cétoamines, les α- et β-hydroxyacides, les aminoacides éventuellement β-hydroxylés, l'acide salicylique et les dérivés de celui-ci. On peut citer en particulier le méthacrylate d'acétoxyéthyle, l'α-hydroxyméthacrylaté de méthyle, l'ε-N-méthacryloyl-L-lysine, l'acide méthacrylamino-4-salicylique, l'acide méthacrylamido-5-salicylique, l'acétoacétate, l'éthylacétoacétate et l'EDTA.

Le ou les composants (A) décrits ci-dessus sont soit sous forme de particules colloïdales soit sous forme de composés solubles qui, après hydrolyse et condensation, donnent des particules nanométriques. Pour pouvoir former le système organo-minéral utilisé dans le procédé de la présente invention, les particules doivent établir des liaisons avec les polymères organiques ou siliconés formant le composant (B). Pour cela elles doivent être capables de réagir et/ou porter au moins un groupement capable de réagir avec ces polymères. Ce groupement peut être porté par un des radicaux organiques R, R' ou R" ou bien par le ligand X ou X' des composés décrits ci-dessus sous les points (2) et (3).
Le substituant capable de réagir avec le polymère organique ou siliconé fonctionnalisé (B) est choisi notamment parmi les radicaux à insaturation éthylénique tels que les groupes (méth)acryliques et vinyliques, les radicaux halogénés, hydroxylés, carboxylés, thiols, époxy, esters, amino, amido, aminoacide, polypeptidique, uréthanne, uréïdo, acétoacétate, éthylacétoacétate ou un groupe dérivant de l'EDTA et les dérivés de celui-ci.

On peut citer à titre d'exemples de composants (A) métallo-organiques préférés de la présente invention le tétraéthoxysilane, l'orthotitanate de tétra-*n*-propyle, l'orthotitanate de tétra-*iso*-propyle, le zirconate de tétra-*n*-propyle, le zirconate de tétra-*iso*-propyle, le méthyltriéthoxysilane, le tétra-éthoxytitane, le tétrabutoxytitane, le triéthoxyfer et le triéthoxytungstène.

Des composés métallo-organiques particulièrement intéressants pour le procédé de la présente invention sont l'orthotitanate de tétra-*iso-*propyle, l'orthotitanaté de tétra-*n*-propyle, le zirconate de tétra-*iso*-propyle et le zirconate de tétra-*n*-propyle.

Il est bien entendu que le composant (A) peut être un mélange de deux ou plusieurs composés métalliques et/ou métallo-organiques.

Ces polymères, lorsqu'ils ne comportent pas en eux-mêmes de groupements fonctionnels capables de réagir avec le composant (A), peuvent être fonctionnalisés dans une étape supplémentaire préalable au mélange avec le composant (A).

Des polymères particulièrement intéressants en tant que composants (B) des matériaux hybrides organo-minéraux utilisés selon la présente invention sont des polydiméthylsiloxanes, de préférence fonctionnalisés, et tout particulièrement des polydiméthylsiloxanes hydroxylées, notamment les PDMS-diol et PDMS-disilanol. On peut également citer des PDMS portant des greffons d'oxyde d'éthylène et/ou d'oxyde de propylène ou des polymères séquencés OE/OP-PDMS-OE/OP.

Comme exemple de précurseur de polymère siliconé utilisable dans le procédé de la présente invention, on peut citer en particulier le diéthyldiméthoxysilane.

Le rapport de mélange du composé métallique ou métallo-organique (A) au polymère organique ou siliconé (B) dans le matériau hybride organo-minéral utilisé selon la présente invention dépend d'un grand nombre de paramètres tels que la nature chimique de ces deux composants, du type de composition cosmétique, du mode d'utilisation du matériau hybride (incorporation dans ou application sur la composition cosmétique), de la quantité de solvant, des propriétés non-transfert recherchées etc.
Le composant métallique ou métallo-organique (A) est généralement présent à raison de 1 à 99 % en poids, de préférence à raison de 5 à 80 % en poids, et tout particulièrement à raison de 10 à 70 % en poids, rapporté au poids du matériau hybride organominéral.
Le composant polymère (B) est généralement présent à raison de 1 à 99 % en poids, de préférence à raison de 5 à 80 % en poids, et tout particulièrement à raison de 10 à 70 % en poids, rapporté au poids du matériau hybride organominéral.

Le solvant (C) éventuellement présent dans le matériau hybride organo-minéral peut être volatil ou non volatil et peut être choisi parmi les alcools, notamment les alcools aliphatiques en C₁₋₆, linéaires ou ramifiés, les huiles siliconées, volatiles ou non volatiles, les huiles hydrocarbonées, volatiles ou non, les huiles végétales, minérales, synthétiques ou animales, ou les mélanges de ces solvants. On peut citer en particulier l'éthanol, les huiles siliconées cycliques ou linéaires comportant de 4 à 12 atomes de silicium, l'huile de ricin, l'huile d'abricot et l'huile de parléam.

Le matériau hybride organo-minéral décrit ci-dessus est utilisé selon la présente invention dans un procédé d'amélioration des propriétés de non-transfert et/ou de brillance d'une composition cosmétique, notamment de soin ou de maquillage, susceptible d'être appliquée sur la peau du corps ou du visage, les lèvres.

Dans ce premier mode de réalisation, on recouvre la composition cosmétique, appliquée sur la peau les lèvres avec ledit matériau hybride organe-minéral, puis on laisse évaporer la fraction volatile de manière à former un film dudit matériau hybride organo-minéral au-dessus de ladite composition cosmétique. Ce film recouvrant et protégeant la composition cosmétique est appelé dans le domaine cosmétique "top coat".

L'invention a également pour objet un procédé pour améliorer les propriétés de non transfert d'un fonds de teint ou d'un rouge à lèvre caractérisée par le fait que l'on applique sur la peau ou les lèvres une position de fond de teint ou un rouge à lèvre dans laquelle on a incoporé un matériau hybride organo-minéral obtenu par voie sol-gel à partir du mélange défini ci dessus

Dans ce deuxième mode de réalisation du procédé de l'invention, on incorpore de façon homogène ledit matériau hybride organo-minéral dans la composition cosmétique de fond de teint ou de rouge à lèvre. Ceci donne une composition cosmétique non-transfert qui peut être appliquée telle quelle sur la peau, les lèvres

Dans ce deuxième mode de réalisation du procédé de la présente invention la quantité de matériau hybride organominéral présent dans la composition cosmétique dépend du type de composition, des propriétés de non-transfert que l'on souhaite obtenir, de la facilité de démaquillage etc.. La fraction du matériau hybride organominéral dans la la composition cosmétique finale obtenue est généralement comprise entre 10 et 90 %, et de préférence entre 15 et 70 % en poids.

Ces compositions peuvent comprendre par ailleurs les ingrédients usuels dans le domaine considéré.

Dans les deux modes de réalisation du procédé de l'invention, le matériau hybride organo-minéral, appliqué avec la composition cosmétique ou au-dessus de celle-ci, forme après évaporation de la fraction volatile une structure réticulée qui confère à la composition cosmétique d'excellentes propriétés de non-transfert.

Le procédé de la présente invention améliore également le confort d'utilisation des compositions cosmétiques. En effet, l'incorporation du matériau hybride dans la composition ou l'application de celui-ci en un revêtement (top-coat) recouvrant la composition ne s'accompagne pas d'une impression d'inconfort rencontrée habituellement pour les compositions non-transfert connues.
Ce procédé se distingue également par la grande brillance qu'il est possible d'obtenir en choisissant de manière adéquate les constituants de départ. Cette propriété est particulièrement recherchée dans le domaine des rouges à lèvres. Lorsque cette propriété est considérée comme indésirable, par exemple dans le domaine des fonds de teint, il est également possible de choisir les constituants de départ de manière à obtenir un dépôt plus ou moins mat.
La présente invention sera décrite plus en détail à l'aide des exemples suivants qui ont un caractère purement illustratif.

### Exemple 1

### Préparation d'un rouge à lèvres non-transfert

On dilue 10,36 g d'un polydiméthylsiloxane-disilanol ayant une masse molaire moyenne en poids égale à 550 avec 4,6 g d'huile de parléam. On ajoute au mélange 6,84 g d'orthotitanate de tétraisopropyle et on maintient le mélange pendant 30 minutes sous agitation ce qui donne le matériau hybride organo-minéral.
Parallèlement, on fait fondre 2,0 g d'une composition de rouge à lèvres commerciale (Rouge Magnétique de Lancôme, teinte *A Vos Amours)* à une température de 80 °C. Après fusion totale, on y ajoute 2 g du matériau hybride organo-minéral préparé ci-dessus et on homogénéise pendant environ 5 minutes. Ensuite, on laisse refroidir la formule à température ambiante. La pâte de rouge à lèvre ainsi obtenue est prête à l'emploi.
La formule obtenue est appliquée sur les lèvres. Le temps de séchage est équivalent à celui d'un rouge à lèvres classique, à savoir d'environ 3 à 10 minutes selon la quantité appliquée.
Le dépôt obtenu se distingue par une meilleure tenue et par un aspect plus brillant qu'un dépôt du rouge à lèvres commercial. Le confort d'utilisation de la formule non-transfert est équivalent à celui-de la formule normale.

### Exemple 2

### Préparation d'un rouge à lèvres non-transfert

On dilue 6,2 g d'un PDMS-disilanol ayant une masse molaire moyenne en poids égale à 4000 avec 2,8 g d'huile de parléam. On ajoute au mélange 9,4 g d'une solution de zirconate de tétra-*n*-propyle à 70 % dans du propanol et on maintient le mélange pendant 30 minutes sous agitation ce qui donne le matériau hybride organo-minéral.
Parallèlement, on fait fondre 2,0 g d'une composition de rouge à lèvres commerciale (Rouge Magnétique de Lancôme, teinte A *Vos Amours*) à une température de 80 °C. Après fusion totale, on y ajoute 2 g du matériau hybride organo-minéral préparé ci-dessus et on homogénéise pendant environ 5 minutes. Ensuite, on laisse refroidir la formule à température ambiante. La pâte de rouge à lèvre ainsi obtenue est prête à l'emploi.

La formule obtenue est appliquée sur les lèvres. Le temps de séchage est équivalent à celui d'un rouge à lèvres classique, à savoir d'environ 3 à 10 minutes selon la quantité appliquée.
Le dépôt obtenu se distingue par une meilleure tenue et par un aspect plus brillant qu'un dépôt du rouge à lèvres commercial. Le confort d'utilisation de la formule non-transfert est équivalent à celui-de la formule normale.

### Exemple 3

### Préparation d'un rouge à lèvres non-transfert

On dilue 6,2 g d'un PDMS-disilanol ayant une masse molaire moyenne en poids égale à 4000 avec 2,8 g d'huile de parléam. On ajoute au mélange 9,4 g d'une solution de zirconate de tétra-n-propyle à 70 % dans du propanol et on maintient le mélange pendant 30 minutes sous agitation ce qui donne le matériau hybride organo-minéral.
Parallèlement, on fait fondre 2,0 g d'une composition de rouge à lèvres commerciale *(Rouge Absolu* de Lancôme) à une température de 80 °C. Après fusion totale, on y ajoute 3 g du matériau hybride organo-minéral préparé ci-dessus et on homogénéise pendant environ 5 minutes. Ensuite, on laisse refroidir la formule à température ambiante. La pâte de rouge à lèvre ainsi obtenue est prête à l'emploi.
La formule obtenue est appliquée sur les lèvres. Le temps de séchage est équivalent à celui d'un rouge à lèvres classique, à savoir d'environ 3 à 10 minutes selon la quantité appliquée.
Le dépôt obtenu se distingue par une meilleure tenue et par un aspect plus brillant qu'un dépôt du rouge à lèvres commercial. Le confort d'utilisation de la formule non-transfert est équivalent à celui-de la formule normale.

### Example 4

### Utilisation d'un revêtement (top-coat) au dessus d'un rouge à lèvres

On dilue 10,4 g de PDMS-disilanol (masse molaire moyenne en poids 550) dans 4,6 g d'huile de parléam puis on y ajoute 6,8 g d'orthotitanate de tétraéthyle. Après avoir agité le mélange pendant environ 30 minutes, on y ajoute 1,3 g de 3-aminopropyltriéthoxysilane et on poursuit l'agitation pendant 5 minutes supplémentaires.
Ce matériau hybride organo-minéral est appliqué avec un pinceau sur des lèvres recouvertes d'un rouge à lèvres commercial (Rouge Magnétique de Lancôme, teinte *A Vos Amours*)*.* Le temps de séchage du revêtement est d'environ 1 à 2 minutes selon la quantité de produit déposée.
Le rouge à lèvres ainsi recouvert d'une couche de matériau hybride présente une excellente tenue et ne se dépose pas sur des objets venant en contact avec les lèvres. Le revêtement protecteur (top-coat) résiste à l'eau. Il confère aux lèvres un aspect plus brillant que le rouge à lèvres seul. La présence du revêtement ne diminue aucunement le confort d'utilisation du rouge à lèvres.

### Exemple 5

### Utilisation d'un revêtement (ton-coat) au dessus d'un fond de teint

On dilue 6,2 g de polydiméthylsiloxane-diol (masse molaire moyenne en poids 4000) dans 2,8 g d'huile de parléam, puis on y ajoute 9,4 g d'une solution de zirconate de tétrapropyle à 70 % dans du propanol. Après 30 minutes d'agitation, le matériau est prêt à être utilisé en tant que top-coat. :
Le top-coat préparé ainsi est appliqué avec une éponge sur le visage recouvert d'un fond de teint commercial (Invisible Perfection de L'Oréal). Le temps de séchage du revêtement est d'environ 1 à 2 minutes selon la quantité de produit déposée.
L'évaluation dans les conditions normales d'utilisation révèle les avantages suivants par rapport au fond de teint normal :
- meilleure tenue
- absence de transfert
- rémanence à l'eau
et le revêtement n'amoindrit en rien le confort d'utilisation du fond de teint.

### Example 6

### Préparation d'un fond de teint non transfert

On dilue 18,7 g d'un PDMS à extrémités oxyéthylénées (masse moyenne en poids = 1500) dans 8,5 g d'huile de parléam. On ajoute au mélange 28,1 g de zirconate de tétra-n-propyle à 70 % dans le propanol, et on maintient le mélange pendant 30 minutes sous agitation.
En mélangeant 2 g de fond de teint cire (Sublime Finish de L'Oréal) et 1 g de matériau hybride préparé ci-dessus on obtient un fond de teint non transfert.
Les propriétés de séchage et le confort d'utilisation sont équivalents à ceux d'un fond de teint normal. Les propriétés de non transfert sont nettement améliorées par rapport au fond de teint d'origine.

## Revendications

1. Procédé pour améliorer les propriétés de non-transfert d'une composition cosmétique appliquée sur la peau et les lèvres **caractérisé par le fait que** que l'on recouvre ladite composition cosmétique par un matériau hybride organo-minéral obtenu par voie sol-gel à partir d'un mélange comprenant :
(A) au moins un composé métallique ou métallo-organique, et
(B) au moins polymère organique ou siliconé choisi parmi un homopolymère ou un copolymère statisticue, séquences et/ou greffé choisi parmi :
(a) les homopolymères et copolymères d'alkyloxazoline ;
(b) les homopolymères et copolymères d'acide (méth)acrylique, d'acide crotonique, d'acide maléique, d'acide itaconique, d'acide styrène-sulfonique, d'acide acrylamido-2-méthylpropane-sulfonique de méthacrylate de 2-sulfoéthyle, d'acide vinylsulfonique et/ou d'acide vinylphosphonique :
(c) les homopolymères d'esters ou d'amides acryliques ou méthacryliques et leurs copolymères avec des comonomères choisis parmi les acides carboxyliques insaturés les acides sulfoniques les acides phosphoniques, les esters et éthers vinyliques, les oléfines, le styrène, les styrènes substitués, les fluoro- ou perfluorooléfines, les (méth)acrylates de perfluoroalkyle les composés vinyliques fluorés et les organosilanes, organosiloxanes ou organopolysiloxanes insaturés ;
(d) les homopolymères et copolymères d'alcool vinylique.
(e) les homopolymères d'esters ou d'amides vinyliques et/ou allyliques et/ou méthallyliques et leurs copolymères avec des comonomères choisis parmi les acides carboxyliques insaturés, les acides sulfoniques, les acides phosphoniques, les esters et éthers vinyliques, les oléfines, le styrène, les styrènes substitués les fluoro- et perfluorooléfines, les (méth)acrylates de perfluoroalkyle, les composés vinyliques fluorés et les organosilanes, organosiloxanes ou organopolysiloxanes insaturés ;
(f) les polyéthers :
(g) les polyesters ;
(h) les homo- et copolymères d'oléfines ou de cyclooléfines ;
(i) les polyamides et polyesteramides :
(j) les polyuréthannes et les polyurées pouvant comporter des séquences polyéther, polyester et/ou polyorganosiloxane ;
(k) les polymères fluorés ;
(l) les polymères naturels et les polymères naturels modifiés (polymères artificiels) ;
(m) les polyorganosiloxanes ;
(n) les polyorganophosphazènes :
(o) les polysilanes -(SiR^{a}R^{b})ₙ, les polycarbosilanes -(SiR^{a}R^{b-}CR^{c}R^{d}-)ₙ et les polysilazanes -(-SiR^{a}R^{b}-NR^{c}-)ₙ, et
(p) des mélanges de ces polymères,
ou un précurseur de ceux-ci
formant après évaporation de la fraction volatile une structure réticulée.

2. Procédé selon la revendication 1, **caractérisé par le fait que** le mélange donnant ledit matériau hybride organo-minéral contient en outre au moins un solvant (composant (C)).

3. Procédé selon l'une quelconque des revendications précédentes, **caractérisé par le fait que** ledit composé (A) est un composé métallique choisi parmi
(i) les oxydes des métaux de transition des groupes IB à VIIB ou du groupe des lanthanides de la classification périodique des éléments,
(ii) les oxydes d'aluminium, de bore, de silicium ou d'étain, et
(iii) les phosphates d'aluminium.

4. Procédé selon la revendication 3, **caractérisé par le fait que** ledit composé (A) est choisi parmi l'oxyde de titane, l'oxyde de fer et l'oxyde de zirconium.

5. Procédé selon la revendication 1 ou 2, **caractérisé par le fait que** le composé (A) est un composé métallo-organique choisi parmi :
(1) les composés correspondant à l'une des formules suivantes :
(Ia) M(OR₁)ₙ
(Ib) R-M-(OR₁)ₙ₋₁
(Ic) (OR₁)ₙ₋₁-M-R"-M(OR₁)ₙ₋₁ 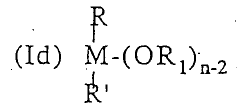
M représente un atome d'un métal de transition des groupes IB à VIIB de la classification périodique des éléments ou un atome d'aluminium, de bore, de silicium ou d'étain, de préférence un atome de Si, Ti ou Zr,
n est égal à la valence de M,
R1 représente un radical alkyle linéaire ou ramifié en C1-30, de préférence en C1-6,
R et R' représentent, indépendamment l'un de l'autre, un groupe alkyle linéaire ou ramifié, un groupe cycloalkyle ou un groupe aryle substitué ou non, lesdits groupes R et R' pouvant eux-mêmes être capable de réagir avec le polymère organique ou siliconé (B) ou pouvant porter un substituant choisi parmi les radicaux à insaturation éthylénique, les radicaux halogénés, hydroxylés, carboxylés thiols, époxy, esters, amino, amido, aminoacide, polypeptidique, uréthane, uréïdo, acétoacétate, éthylacétoacétate, ou un groupe dérivant de l'EDTA et les dérivés de celui-ci,
R" représente un radical alkylène linéaire ou ramifié, cycloalkylène ou arylène éventuellement substitué, pouvant réagir lui-même avec le polymère organique ou siliconé (B) ou pouvant porter un substituant choisi parmi les radicaux à insaturation éthylénique, les radicaux halogénés, hydroxylés, carboxylés thiols, époxy, esters amino, amido, aminoacide, polypeptidique uréthane, uréïdo, acétoacétate, éthylacétoacétate ou un groupe dérivant de l'EDTA et les dérivés de celui-ci,
(2) les complexes de coordination des composés de formule (Ia) à (Id) ci-dessus, correspondant à une des formules suivantes
(IIa) M(OR₁)ₙ₋ₓ (X)ₓ
(IIb) R-M(OR₁)ₙ₋₁₋ₓ (X)ₓ
(IIc) (X)ₓ (OR₁)ₙ₋₁₋ₓM-R"-M-(OR₁)ₙ₋₁₋ₓ (X)ₓ 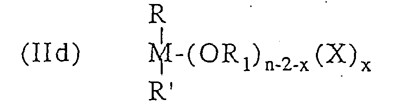
M représente un atome d'un métal de transition des groupes IB à VIIB de la classification périodique des éléments ou un atome d'aluminium, de bore, de silicium ou d'étain, de préférence un atome de Ti ou Zr,
X est un ligand monocoordiné ou monodenté comportant un atome d'azote, un atome de phosphore, un atome de soufre ou un atome d'oxygène, et pouvant porter un substituant choisi parmi les radicaux à insaturation éthylénique, les radicaux halogénés, hydroxylés, carboxylés, thiols, époxy, esters, amino, amido, aminoacide, polypeptidique, uréthane, uréïdo, acétoacétate, éthylacétoacétate ou un groupe dérivant de l'EDTA et les dérivés de celui-ci, et pouvant porter un groupe choisi parmi les radicaux à insaturation éthylénique, les radicaux halogénés, hydroxylés, carboxylés, thiols, époxy, esters, amino, amido, aminoacide, polypeptidique, uréthane, uréïdo acétoacétate, éthylacétoacétate ou un groupe dérivant de l'EDTA et les dérivés de celui-ci,
x représente le nombre de ligands X, et
n, R, R₁, R' et R" sont tels que définis pour les formules (Ia) à (Id), et
(3) les complexes de chélation répondant à la formule suivante :
(III) M(OR₁)_{n-bx} (X')ₓ
dans laquelle
M représente un atome d'un métal de transition des groupes IB à VIIB de la classification périodique des éléments ou un atome d'aluminium, de bore, de silicium ou d'étain, de préférence un atome de Ti ou Zr,
X' représente un groupe chélatant ou ligand polydenté comportant un atome d'azote, un atome de phosphore, un atome de soufre et/ou un atome d'oxygène, et pouvant porter un substituant choisi parmi les radicaux à insaturation éthylénique, les radicaux halogènes, hydroxylés, carboxylés, thiols, époxy, esters, amino amido aminoacide, polypeptidique, uréthane uréïdo, acétoacétate, éthylacétoacétate ou un groupe dérivant de l'EDTA et les dérivés de celui-ci, et pouvant porter un groupe choisi parmi les radicaux à insaturation éthylénique, les radicaux halogénés, hydroxylés, carboxylés, thiols, époxy, esters*,* amino, amido, aminoacide polypeptidique, uréthane, uréïdo, acétoacétate éthylacétoacétate ou un groupe dérivant de l'EDTA et les dérivés de celui-ci.
b correspond à la valence de coordination du ligand X' et est au moins égal à 2,
x est égal au nombre de ligands X', et
R₁ et n ont les significations indiquées ci-dessus pour les formules (Ia) à (Id).

6. Procédé selon la revendication 5, **caractérisé par le fait que** le ligand monocoordiné X est choisi parmi les acides carboxyliques et les cétones.

7. Procédé selon la revendication 5, **caractérisé par le fait que** le ligand polydenté X' est choisi parmi les β-dicétones, les β-cétoesters, les β-cétoamines, les α- et β-hydroxyacides, les aminoacides éventuellement β-hydroxylés, l'acide salicylique et les dérivés de celui-ci.

8. Procédé selon la revendication 7, **caractérisé par le fait que** le ligand polydenté X' est choisi parmi le méthacrylate d'acétoxyéthyle, l'α-hydroxyméthacrylate de méthyle, l'ε-N-méthacryloyl-L-lysine, l'acide méthacrylamino-4-salicylique et l'acide méthacrylamino-5-salicylique.

9. Procédé selon l'une quelconque des revendications 1 à 2 et 5 à 8, **caractérisé par le fait que** ledit composé métallo-organique est choisi dans le groupe formé par le tétraéthoxysilane, l'orthotitanate de tétra-*n*-propyle, l'orthotitanate de tétra-*iso*-propyle, le zirconate de tétra-*n-*propyle, le zirconate de tétra-*iso*-propyle, le méthyltriéthoxysilane, le tétra-éthoxytitane, le tétrabutoxytitane, le triéthoxyfer et le triéthoxytungstène.

10. Procédé selon la revendication 9, **caractérisé par le fait que** ledit composé métallo-organique est l'orthotitanate de tétra-*iso*-propyle, l'orthotitanate de tétra-*n*-propyle, le zirconate de tétra-*iso*-propyle et le zirconate de tétra-*n*-propyle.

11. Procédé selon l'une quelconque des revendications 1 à 10, **caractérisé par le fait que** le composant métallique ou métallo-organique (A) est présent à raison de 1 à 99 % en poids, de préférence à raison de 5 à 80 % en poids, et tout particulièrement à raison de 10 à 70 % en poids, rapporté au poids du matériau hybride organominéral.

12. Procédé pour améliorer les propriétés de non transfert d'un fonds de teint ou d'un rouge à lèvre **caractérisée par le fait que** l'on applique-sur la peau ou les lèvres une composition de fond de teint ou un rouge à lèvre dans laquelle on a incorporé un matériau hybride organo-minéral obtenu par voie sol-gel à partir d'un mélange comprenant :
(A) au moins un composé métallique ou métallo-organique, et
(B) au moins polymère organique ou siliconé choisi parmi un homopolymère ou un copolymère statistique, séquences et/ou greffé choisi parmi :
(a) les homopolymères et copolymères d'alkyloxazoline :
(b) les homopolymères et copolymères d'acide (méth)acrylique, d'acide crotonique, d'acide maléique, d'acide itaconique, d'acide styrène-sulfonique, d'acide acrylamido-2-méthylpropane-sulfonique, de méthacrylate de 2-sulfoéthyle, d'acide vinylsulfonique et/ou d'acide vinylphosphonique ;
(c) les homopolymères d'esters ou d'amides acryliques ou méthacryliques et leurs copolymères avec des comonomères choisis parmi les acides carboxyliques insaturés les acides sulfoniques les acides phosphoniques, les esters et éthers vinyliques les oléfines, le styrène, les styrènes substitués, les fluoro- ou perfluorooléfines, les (méth)acrylates de perfluoroalkyle, les composés vinyliques fluorés et les organosilanes, organosiloxanes ou organopolysiloxanes insaturés :
(d) les homopolymères et copolymères d'alcool vinylique.
(e) les homopolymères d'esters ou d'amides vinyliques et/ou allyliques et/ou méthallyliques et leurs copolymères avec des comonomères choisis parmi les acides carboxyliques insaturés, les acides sulfoniques; les acides phosphoniques, les esters et éthers vinyliques, les oléfines, le styrène, les styrènes substitués les fluoro- et perfluorooléfines, les (méth)acrylates de perfluoroalkyle, les composés vinyliques fluorés et les organosilanes, organosiloxanes ou organopolysiloxanes insaturés :
(f) les polyéthers ;
(g) les polyesters ;
(h) les homo- et copolymères d'oléfines ou de cyclooléfines :
(i) les polyamides et polyesteramides
(j) les polyuréthannes et les polyurées pouvant comporter des séquences polyéther, polyester et/ou polyorganosiloxane :
(k) les polymères fluorés :
(l) les polymères naturels et les polymères naturels modifiés (polymères artificiels) :
(m) les polyorganosiloxanes ;
(n) les polyorganophosphazènes ;
(o) les polysilanes -(SiR^{a}R^{b}-)n les polycarbosilanes -(SiR^{a}R^{b-}CR^{c}R^{d}-)ₙ et les polysilazanes -(-SiR^{a}R^{b}-NR^{c}-)ₙ, et
(p) des mélanges de ces polymères,
ou un précurseur de ceux-ci.
ledit matériau hybride formant après évaporation de la fraction volatile une structure réticulée.

13. Procédé selon la revendication 12, **caractérisé par le fait que** le mélange donnant ledit matériau hybride organo-minéral contient en outre au moins un solvant (composant (C)).

14. Procédé selon la revendication 12 ou 13, **caractérisé par le fait que** l'on incorpore de façon homogène ledit matériau hybride organo-minéral dans la composition cosmétique.

15. Procédé selon l'une quelconque des revendications 12 à 14, **caractérisé par le fait que** ledit composé (A) est un composé métallique choisi parmi
(i) les oxydes des métaux de transition des groupes IB à VIIB ou du groupe des lanthanides de la classification périodique des éléments,
(ii) les oxydes d'aluminium, de bore, de silicium ou d'étain, et
(iii) les phosphates d'aluminium.

16. Procédé selon la revendication 15, **caractérisé par le fait que** ledit composé (A) est choisi parmi l'oxyde de titane, l'oxyde de fer et l'oxyde de zirconium.

17. Procédé selon l'une quelconque des revendications 12 à 14, **caractérisé par le fait que** le composé (A) est un composé métallo-organique choisi parmi :
(1) les composés correspondant à l'une des formules suivantes :
(Ia) M(OR₁)ₙ
(Ib) R-M-(OR₁)ₙ₋₁
(Ic) (OR₁)ₙ₋₁-M-R"-M(OR₁)ₙ₋₁ 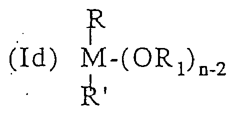
M représente un atome d'un métal de transition des groupes IB à VIIB de la classification périodique des éléments ou un atome d'aluminium, de bore, de silicium ou d'étain, de préférence un atome de Si, Ti ou Zr,
n est égal à la valence de M,
R1 représente un radical alkyle linéaire ou ramifié en C1-30, de préférence en C1-6,
R et R' représentent, indépendamment l'un de l'autre, un groupe alkyle linéaire ou ramifié, un groupe cycloalkyle ou un groupe aryle substitué ou non, lesdits groupes R et R' pouvant eux-mêmes être capable de réagir avec le polymère organique ou siliconé (B) ou pouvant porter un substituant choisi parmi les radicaux à insaturation éthylénique, les radicaux halogénés, hydroxylés, carboxylés, thiols, époxy, esters, amino, amido, aminoacide, polypeptidique, uréthane, uréïdo, acétoacétate éthylacétoacétate ou un groupe dérivant de l'EDTA et les dérivés de celui-ci.
R" représente un radical alkylène linéaire ou ramifié, cycloalkylène ou arylène éventuellement substitué, pouvant réagir lui-même avec le polymère organique ou siliconé (B) ou pouvant porter un substituant choisi parmi les radicaux à insaturation éthylénique, les radicaux halogénés, hydroxylés carboxylés, thiols, époxy, esters, amino, amido, aminoacide, polypeptidique, uréthane, uréïdo, acétoacétate. éthylacétoacétate ou un groupe dérivant de l'EDTA et les dérivés de celui-ci.
(2) les complexes de coordination des composés de formule (Ia) à (Id) ci-dessus, correspondant à une des formules suivantes
(IIa) M(OR₁)ₙ₋ₓ (X)ₓ
(IIb) R-M(OR₁)ₙ₋₁₋ₓ (X)ₓ
(IIC) (X)ₓ (OR₁)ₙ₋₁₋ₓM-R"-M-(OR₁)ₙ₋₁₋ₓ (X)ₓ 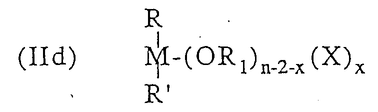
M représente un atome d'un métal de transition des groupes IB à VIIB de la classification périodique des éléments ou un atome d'aluminium, de bore, de silicium ou d'étain, de préférence un atome de Ti ou Zr,
X est un ligand monocoordiné ou monodenté comportant un atome d'azote, un atome de phosphore, un atome de soufre ou un atome d'oxygène, et pouvant porter un substituant choisi parmi les radicaux à insaturation éthylénique, les radicaux halogénés, hydroxylés carboxylés thiols époxy esters, amino, amido, aminoacide, polypeptidique, uréthane uréïdo acétoacétate. éthylacétoacétate ou un groupe dérivant de l'EDTA et les dérivés de celui-ci, et pouvant porter un groupe choisi parmi les radicaux à insaturation éthylénique, les radicaux halogénés hydroxylés, carboxylés thiols époxy esters, amino amido aminoacide, polypeptidique, uréthane uréïdo acétoacétate, éthylacétoacétate ou un groupe dérivant de l'EDTA et les dérivés de celui-ci
x représente le nombre de ligands X, et
n, R, R₁, R' et R" sont tels que définis pour les formules (Ia) à (Id), et
(3) les complexes de chélation répondant à la formule suivante :
(III) M(OR₁)_{n-bx} (X')ₓ
dans laquelle
M représente un atome d'un métal de transition des groupes IB à VIIB de la classification périodique des éléments ou un atome d'aluminium, de bore, de silicium ou d'étain, de préférence un atome de Ti ou Zr,
X' représente un groupe chélatant ou ligand polydenté comportant un atome d'azote, un atome de phosphore, un atome de soufre et/ou un atome d'oxygène, et pouvant porter un substituant choisi parmi les radicaux à insaturation éthylénique, les radicaux halogénés. h^{y}droxylés, carboxylés, thiols, époxy, esters, amino, amido, aminoacide, polypeptidique, uréthane, uréïdo, acétoacétate. éthylacétoacétate ou un groupe dérivant de l'EDTA et les dérivés de celui-ci, et pouvant porter un groupe choisi parmi les radicaux à insaturation éthylénique, les radicaux halogénés, hydroxylés. carboxylés, thiols, époxy, esters, amino, amido, aminoacide, polypeptidique, uréthane, uréïdo, acétoacétate, éthylacétoacétate ou un groupe dérivant de l'EDTA et les dérivés de celui-ci,
b correspond à la valence de coordination du ligand X' et est au moins égal à 2,
x est égal au nombre de ligands X', et
R₁ et n ont les significations indiquées ci-dessus pour les formules (Ia) à (Id).

18. Procédé selon la revendication 17, **caractérisé par le fait que** le ligand monocoordiné X est choisi parmi les acides carboxyliques et les cétones.

19. Procédé selon la revendication 17, **caractérisé par le fait que** le ligand polydenté X' est choisi parmi les β-dicétones, les β-cétoesters, les β-cétoamines, les α- et β-hydroxyacides, les aminoacides éventuellement β-hydroxylés, l'acide salicylique et les dérivés de celui-ci.

20. Procédé selon la revendication 19, **caractérisé par le fait que** le ligand polydenté X' est choisi parmi le méthacrylate d'acétoxyéthyle, l'α-hydroxyméthacrylate de méthyle, l'ε-N-méthacryloyl-L-lysine, l'acide méthacrylamino-4-salicylique et l'acide méthacrylamino-5-salicylique.

21. Procédé selon l'une quelconque des revendications 12 à 14 et 17 à 20, **caractérisé par le fait que** ledit composé métallo-organique est choisi dans le groupe formé par le tétraéthoxysilane, l'orthotitanate de tétra-*n*-propyle, l'orthotitanate de tétra-*iso*-propyle, le zirconate de tétra-*n*-propyle, le zirconate de tétra-*iso*-propyle, le méthyltriéthoxysilane, le tétra-éthoxytitane, le tétrabutoxytitane, le triéthoxyfer et le triéthoxytungstène.

22. Procédé selon la revendication 21, **caractérisé par le fait que** ledit composé métallo-organique est l'orthotitanate de tétra-*iso*-propyle, l'orthotitanate de tétra-*n*-propyle, le zirconate de tétra-*iso*-propyle et le zirconate de tétra-*n*-propyle.

23. Procédé selon l'une quelconque des revendications 12 à 22, **caractérisé par le fait que** le composant métallique ou métallo-organique (A) est présent à raison de 1 à 99 % en poids, de préférence à raison de 5 à 80 % en poids, et tout particulièrement à raison de 10 à 70 % en poids, rapporté au poids du matériau hybride organominéral.

24. Procédé selon la revendication l'une quelconque des revendications 12 à 23, **caractérisé par le fait que** l'on incorpore ledit matériau hybride organo-minéral à raison de 10 à 90 % en poids, de préférence à raison de 15 à 70 % en poids; rapporté au poids total de la composition cosmétique finale.

25. Procédé selon l'une quelconque des revendications précédentes **caractérisé par le fait que** les polyorganosiloxanes sont les polydiméthylsiloxanes, les polydiméthylsiloxanes hydroxylés, les polydiméthylsiloxanes portant des greffons d'oxyde d'éthylène et/ou d'oxyde de propylène oui des polymères séquencés OE/OP-PDMS-OE/OP.

26. Procédé selon la revendication 25, **caractérisé par le fait que** ledit polymère organique ou siliconé (B) est un polydiméthylsiloxane hydroxylé.

27. Procédé selon l'une quelconque des revendications 2 à 26, **caractérisé par le fait que** ledit solvant est choisi parmi les alcools, notamment les alcools aliphatiques en C₁₋₆, linéaires ou ramifiés, les huiles siliconées volatiles ou non volatiles, les huiles hydrocarbonées volatiles ou non, les huiles végétales, minérales, synthétiques ou animales, ou les mélanges de ceux-ci.

28. Procédé selon l'une quelconque des revendications précédentes, **caractérisé par le fait que** le composant polymère (B) est généralement présent à raison de 1 à 99 % en poids, de préférence à raison de 5 à 80 % en poids, et tout particulièrement à raison de 10 à 70 % en poids, rapporté au poids du matériau hybride organominéral.

29. Procédé selon l'une quelconque des revendications précédentes, **caractérisé par le fait que** ladite composition cosmétique est une composition de soin ou de maquillage susceptible d'être appliquée sur la peau ou les lèvres.

30. Procédé selon la revendication 29, **caractérisé par le fait que** ladite composition de maquillage est un fond de teint ou un rouge à lèvres.

## Claims

1. Process for improving the transfer-resistance properties of a cosmetic composition applied to the skin and the lips, **characterized in that** the said cosmetic composition is covered with an organo-mineral hybrid material obtained via a sol-gel route, starting with a mixture comprising:
(A) at least one metallic or organometallic compound, and
(B) at least one organic or silicone polymer chosen from a random, block and/or grafted homopolymer or copolymer chosen from:
(a) alkyloxazoline homopolymers and copolymers;
(b) homopolymers and copolymers of (meth)-acrylic acid, of crotonic acid, of maleic acid, of itaconic acid, of styrenesulphonic acid, of acrylamido-2-methylpropanesulphonic acid, of 2-sulphoethyl methacrylate, of vinylsulphonic acid and/or of vinylphosphonic acid;
(c) homopolymers of acrylic or methacrylic esters or amides and copolymers thereof with comonomers chosen from unsaturated carboxylic acids, sulphonic acids, phosphonic acids, vinyl esters and ethers, olefins, styrene, substituted styrenes, fluoroolefins, perfluoroolefins, perfluoroalkyl (meth)acrylates, fluorovinyl compounds and unsaturated organosilanes, organosiloxanes or polyorganosiloxanes;
(d) vinyl alcohol homopolymers and copolymers,
(e) homopolymers of vinyl and/or allylic and/or methallylic esters or amides and copolymers thereof with comonomers chosen from unsaturated carboxylic acids, sulphonic acids, phosphonic acids, vinyl esters and ethers, olefins, styrene, substituted styrenes, fluoroolefins, perfluoroolefins, perfluoroalkyl (meth)acrylates, fluorovinyl compounds and unsaturated organosilanes, organosiloxanes or polyorganosiloxanes;
(f) polyethers;
(g) polyesters;
(h) olefin or cycloolefin homopolymers and copolymers;
(i) polyamides and polyesteramides;
(j) polyurethanes and polyureas which may comprise polyether, polyester and/or polyorganosiloxane sequences;
(k) fluoropolymers;
(l) natural polymers and modified natural polymers (artificial polymers);
(m) polyorganosiloxanes;
(n) polyorganophosphazenes;
(o) polysilanes -(SiR^{a}R^{b}-)ₙ, polycarbosilanes - (SiR^{a}R^{b}-CR^{c}R^{d}-)ₙ and polysilazanes -(SiR^{a}R^{b}-NR^{c}-)ₙ, and
(p) mixtures of these polymers,
or a precursor thereof,
forming a crosslinked structure after evaporation of the volatile fraction.

2. Process according to Claim 1, **characterized in that** the mixture giving the said organo-mineral hybrid material also contains at least one solvent (component (C)).

3. Process according to any one of the preceding claims, **characterized in that** the said compound (A) is a metallic compound chosen from
(i) oxides of transition metals from groups IB to VIIB or from the lanthanide group of the Periodic Table of the Elements,
(ii) aluminium oxide, boron oxide, silicon oxide or tin oxide, and
(iii) aluminium phosphates.

4. Process according to Claim 3, **characterized in that** the said compound (A) is chosen from titanium oxide, iron oxide and zirconium oxide.

5. Process according to either of Claims 1 and 2, **characterized in that** the compound (A) is an organometallic compound chosen from:
(1) compounds corresponding to one of the following formulae:
(Ia) M(OR₁)ₙ
(Ib) R-M-(OR₁)ₙ₋₁
(Ic) (OR₁)ₙ₋₁-M-R"-M(OR₁)ₙ₋₁
and 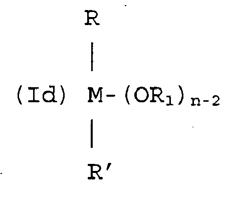 M represents an atom of a transition metal from groups IB to VIIB of the Periodic Table of the Elements or an aluminium, boron, silicon or tin atom, preferably an Si, Ti or Zr atom,
n is equal to the valency of M,
R₁ represents a linear or branched C₁₋₃₀ and preferably C₁₋₆ alkyl radical,
R and R' represent, independently of each other, a linear or branched alkyl group, a cycloalkyl group or a substituted or unsubstituted aryl group, the said groups R and R' themselves possibly being capable of reacting with the organic or silicone polymer (B) or possibly bearing a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
R" represents a linear or branched alkylene, cycloalkylene or arylene radical which is optionally substituted, which may itself react with the organic or silicone polymer (B) or may-bear a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
(2) coordination complexes of the compounds of formulae (Ia) to (Id) above, corresponding to one of the following formulae
(IIa) M(OR₁)ₙ₋ₓ (X)ₓ
(IIb) R-M(OR₁)ₙ₋₁₋ₓ (X)ₓ
(IIc) (X)ₓ (OR₁)ₙ₋₁₋ₓM-R"-M-(OR₁)ₙ₋₁₋ₓ (X)ₓ 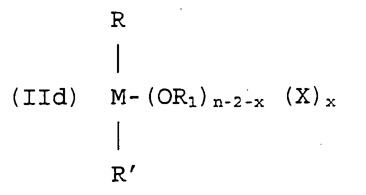
M represents an atom of a transition metal from groups IB to VIIB of the Periodic Table of the Elements or an aluminium, boron, silicon or tin atom and preferably a Ti or Zr atom,
X is a monocoordinate or monodentate ligand comprising a nitrogen atom, a phosphorus atom, a sulphur atom or an oxygen atom, and possibly bearing a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof, and possibly bearing a group chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
x represents the number of ligands X, and
n, R, R₁, R' and R" are as defined for formulae (Ia) to (Id) ; and
(3) chelation complexes corresponding to the following formula:
(III) M(OR₁)_{n-bx} (X')ₓ
in which
M represents an atom of a transition metal from groups IB to VIIB of the Periodic Table of the Elements or an aluminium, boron, silicon or tin atom and preferably a Ti or Zr atom,
X' represents a polydentate ligand or chelating group comprising a nitrogen atom, a phosphorus atom, a sulphur atom and/or an oxygen atom, and possibly bearing a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof, and possibly bearing a group chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
b corresponds to the coordination valency of the ligand X' and is at least equal to 2,
x is equal to the number of ligands X', and
R₁ and n have the meanings given above for formulae (Ia) to (Id).

6. Process according to Claim 5, **characterized in that** the monocoordinate ligand X is chosen from carboxylic acids and ketones.

7. Process according to Claim 5, **characterized in that** the polydentate ligand X' is chosen from β-diketones, β-keto esters, β-keto amines, α-hydroxy acids and β-hydroxy acids, optionally β-hydroxylated amino acids, and salicylic acid and its derivatives.

8. Process according to Claim 7, **characterized in that** the polydentate ligand X' is chosen from acetoxyethyl methacrylate, methyl α-hydroxymethacrylate, ε-N-methacryloyl-L-lysine, methacrylamino-4-salicylic acid and methacrylamino-5-salicylic acid.

9. Process according to any one of Claims 1 to 2 and 5 to 8, **characterized in that** the said organometallic compound is chosen from the group formed by tetraethoxysilane, tetra-n-propyl orthotitanate, tetraisopropyl orthotitanate, tetra-n-propyl zirconate, tetraisopropyl zirconate, methyltriethoxysilane, tetraethoxytitanium, tetrabutoxytitanium, triethoxyiron and triethoxytungsten.

10. Process according to Claim 9, **characterized in that** the said organometallic compound is tetraisopropyl orthotitanate, tetra-n-propyl orthotitanate, tetraisopropyl zirconate or tetra-n-propyl zirconate.

11. Process according to any one of Claims 1 to 10, **characterized in that** the metallic or organometallic component (A) is present in a proportion of from 1% to 99% by weight, preferably in a proportion of from 5% to 80% by weight and most particularly in a proportion of from 10% to 70% by weight, relative to the weight of the organo-mineral hybrid material.

12. Process for improving the transfer-resistance properties of a foundation or a lipstick,
**characterized in that** a foundation composition or a lipstick into which has been incorporated an organo-mineral hybrid material obtained via a sol-gel route starting with a mixture comprising:
(A) at least one metallic or organometallic compound, and
(B) at least one organic or silicone polymer chosen from a random, block and/or grafted homopolymer or copolymer chosen from:
(a) alkyloxazoline homopolymers and copolymers;
(b) homopolymers and copolymers of (meth)-acrylic acid, of crotonic acid, of maleic acid, of itaconic acid, of styrenesulphonic acid, of acrylamido-2-methylpropanesulphonic acid, of 2-sulphoethyl methacrylate, of vinylsulphonic acid and/or of vinylphosphonic acid;
(c) homopolymers of acrylic or methacrylic esters or amides and copolymers thereof with comonomers chosen from unsaturated carboxylic acids, sulphonic acids, phosphonic acids, vinyl esters and ethers, olefins, styrene, substituted styrenes, fluoroolefins, perfluoroolefins, perfluoroalkyl (meth)acrylates, fluorovinyl compounds and unsaturated organosilanes, organosiloxanes or polyorganosiloxanes;
(d) vinyl alcohol homopolymers and copolymers,
(e) homopolymers of vinyl and/or allylic and/or methallylic esters or amides and copolymers thereof with comonomers chosen from unsaturated carboxylic acids, sulphonic acids, phosphonic acids, vinyl esters and ethers, olefins, styrene, substituted styrenes, fluoroolefins, perfluoroolefins, perfluoroalkyl (meth)acrylates, fluorovinyl compounds and unsaturated organosilanes, organosiloxanes or polyorganosiloxanes;
(f) polyethers;
(g) polyesters;
(h) olefin or cycloolefin homopolymers and copolymers;
(i) polyamides and polyesteramides;
(j) polyurethanes and polyureas which may comprise polyether, polyester and/or polyorganosiloxane sequences;
(k) fluoropolymers;
(l) natural polymers and modified natural polymers (artificial polymers);
(m) polyorganosiloxanes;
(n) polyorganophosphazenes;
(o) polysilanes -(SiR^{a}R^{b}-)ₙ, polycarbosilanes - (SiR^{a}R^{b}-CR^{c}R^{d}-)ₙ and polysilazanes - (SiR^{a}R^{b}-NR^{c}-)ₙ, and
(p) mixtures of these polymers, or a precursor thereof,
the said hybrid material forming a crosslinked structure after evaporation of the volatile fraction,
is applied to the skin or the lips.

13. Process according to Claim 12, **characterized in that** the mixture giving the said organo-mineral hybrid material also contains at least one solvent (component (C)).

14. Process according to either Claim 12 or 13, **characterized in that** the said organo-mineral hybrid material is incorporated uniformly into the cosmetic composition.

15. Process according to any one of Claims 12 to 14, **characterized in that** the said compound (A) is a metallic compound chosen from
(i) oxides of transition metals from groups IB to VIIB or from the lanthanide group of the Periodic Table of the Elements,
(ii) aluminium oxide, boron oxide, silicon oxide or tin oxide, and
(iii) aluminium phosphates.

16. Process according to Claim 15, **characterized in that** the said compound (A) is chosen from titanium oxide, iron oxide and zirconium oxide.

17. Process according to any one of Claims 12 to 14, **characterized in that** the compound (A) is an organometallic compound chosen from:
(1) compounds corresponding to one of the following formulae:
(Ia) M(OR₁)ₙ
(Ib) R-M-(OR₁)ₙ₋₁
(Ic) (OR₁)ₙ₋₁-M-R"-M(OR₁)ₙ₋₁
and 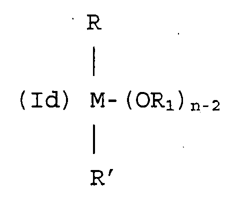 M represents an atom of a transition metal from groups IB to VIIB of the Periodic Table of the Elements or an aluminium, boron, silicon or tin atom, preferably an Si, Ti or Zr atom,
n is equal to the valency of M,
R₁ represents a linear or branched C₁₋₃₀ and preferably C₁₋₆ alkyl radical,
R and R' represent, independently of each other, a linear or branched alkyl group, a cycloalkyl group or a substituted or unsubstituted aryl group, the said groups R and R' themselves possibly being capable of reacting with the organic or silicone polymer (B) or possibly bearing a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
R" represents a linear or branched alkylene, cycloalkylene or arylene radical which is optionally substituted, which may itself react with the organic or silicone polymer (B) or may bear a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
(2) coordination complexes of the compounds of formulae (Ia) to (Id) above, corresponding to one of the following formulae
(IIa) M (OR₁)ₙ₋ₓ (X)ₓ
(IIb) R-M(OR₁)ₙ₋₁₋ₓ (X)ₓ
(IIC) (X)ₓ (OR₁)ₙ₋₁₋ₓM-R"-M-(OR₁)ₙ₋₁₋ₓ (X)ₓ 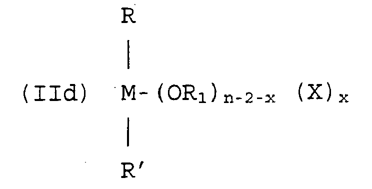
M represents an atom of a transition metal from groups IB to VIIB of the Periodic Table of the Elements or an aluminium, boron, silicon or tin atom and preferably a Ti or Zr atom,
X is a monocoordinate or monodentate ligand comprising a nitrogen atom, a phosphorus atom, a sulphur atom or an oxygen atom, and possibly bearing a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof, and possibly bearing a group chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
x represents the number of ligands X, and
n, R, R₁, R' and R" are as defined for formulae (Ia) to (Id) ; and
(3) chelation complexes corresponding to the following formula:
(III) M(OR₁)_{n-bx} (X')ₓ
in which
M represents an atom of a transition metal from groups IB to VIIB of the Periodic Table of the Elements or an aluminium, boron, silicon or tin atom and preferably a Ti or Zr atom,
X' represents a polydentate ligand or chelating group comprising a nitrogen atom, a phosphorus atom, a sulphur atom and/or an oxygen atom, and possibly bearing a substituent chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof, and possibly bearing a group chosen from radicals containing ethylenic unsaturation, halogenated, hydroxylated, carboxylated, thiol, epoxy, ester, amino, amido, amino acid, polypeptide, urethane, ureido, acetoacetate and ethylacetoacetate radicals, or a group derived from EDTA and derivatives thereof,
b corresponds to the coordination valency of the ligand X' and is at least equal to 2,
x is equal to the number of ligands X', and
R₁ and n have the meanings given above for formulae (Ia) to (Id).

18. Process according to Claim 17, **characterized in that** the monocoordinate ligand X is chosen from carboxylic acids and ketones.

19. Process according to Claim 17, **characterized in that** the polydentate ligand X' is chosen from β-diketones, β-keto esters, β-keto amines, α-hydroxy acids and β-hydroxy acids, optionally β-hydroxylated amino acids, and salicylic acid and its derivatives.

20. Process according to Claim 19, **characterized in that** the polydentate ligand X' is chosen from acetoxyethyl methacrylate, methyl α-hydroxymethacrylate, ε-N-methacryloyl-L-lysine, methacrylamino-4-salicylic acid and methacrylamino-5-salicylic acid.

21. Process according to any one of Claims 12 to 14 and 17 to 20, **characterized in that** the said organometallic compound is chosen from the group formed by tetraethoxysilane, tetra-n-propyl orthotitanate, tetraisopropyl orthotitanate, tetra-n-propyl zirconate, tetraisopropyl zirconate, methyltriethoxysilane, tetraethoxytitanium, tetrabutoxytitanium, triethoxyiron and triethoxytungsten.

22. Process according to Claim 21, **characterized in that** the said organometallic compound is tetraisopropyl orthotitanate, tetra-n-propyl orthotitanate, tetraisopropyl zirconate or tetra-n-propyl zirconate.

23. Process according to any one of Claims 12 to 22, **characterized in that** the metallic or organometallic component (A) is present in a proportion of from 1% to 99% by weight, preferably in a proportion of from 5% to 80% by weight and most particularly in a proportion of from 10% to 70% by weight, relative to the weight of the organo-mineral hybrid material.

24. Process according to any one of Claims 12 to 23, **characterized in that** the said organo-mineral hybrid material is incorporated in a proportion of from 10% to 90% by weight and preferably in a proportion of from 15% to 70% by weight relative to the total weight of the final cosmetic composition.

25. Process according to any one of the preceding claims, **characterized in that** the polyorganosiloxanes are polydimethylsiloxanes, hydroxylated polydimethylsiloxanes, polydimethylsiloxanes bearing ethylene oxide and/or propylene oxide grafts or EO/PO-PDMS-EO/PO block polymers.

26. Process according to Claim 25, **characterized in that** the said organic or silicone polymer (B) is a hydroxylated polydimethylsiloxane.

27. Process according to any one of Claims 2 to 26, **characterized in that** the said solvent is chosen from alcohols, in particular linear or branched C₁₋₆ aliphatic alcohols, volatile or non-volatile silicone oils, volatile or non-volatile hydrocarbon-based oils and plant, mineral, synthetic or animal oils, or mixtures thereof.

28. Process according to any one of the preceding claims, **characterized in that** the polymeric component (B) is generally present in a proportion of from 1% to 99% by weight, preferably in a proportion of from 5% to 80% by weight and most particularly in a proportion of from 10% to 70% by weight, relative to the weight of the organo-mineral hybrid material.

29. Process according to any one of the preceding claims, **characterized in that** the said cosmetic composition is a care or make-up composition which may be applied to the skin or the lips.

30. Process according to Claim 29, **characterized in that** the said make-up composition is a foundation or a lipstick.

## Patentansprüche

1. Verfahren zur Verbesserung der Abriebfestigkeit einer kosmetischen Zusammensetzung, die auf die Haut und die Lippen aufgetragen wird, **dadurch gekennzeichnet, dass** die kosmetische Zusammensetzung mit einem organisch-anorganischen Hybridmaterial bedeckt wird, das durch ein Sol-Gel-Verfahren aus einem Gemisch hergestellt wird, das enthält:
(A) mindestens eine metallische oder Metall-organische Verbindung und
(B) mindestens ein organisches oder siliconiertes Polymer, das unter einem statistischen, sequentiellen und/oder gepfropften Homopolymer oder Copolymer ausgewählt ist, welches ausgewählt ist unter:
(a) Alkyloxazolin-Homopolymeren und Alkyloxazolin-Copolymeren;
(b) Homopolymeren und Copolymeren von (Meth)acrylsäure, Crotonsäure, Maleinsäure, Itaconsäure, Styrol-sulfonsäure, Acrylamido-2-methylpropan-sulfonsäure, 2-Sulfoethylmethacrylat, Vinylsulfonsäure und/oder Vinylphosphonsäure;
(c) Homopolymeren von Acrylestern, Methacrylestern, Acrylamiden oder Methacrylamiden und deren Copolymeren mit Comonomeren, die unter ungesättigten Carbonsäuren, Sulfonsäuren, Phosphonsäuren, Vinylestern und Vinylethern, Olefinen, Styrol, substituierten Styrolen, Fluor- oder Perfluorolefinen, Perfluoralkyl(meth)acrylaten, fluorierten Vinylverbindungen und Organosilanen, Organosiloxanen oder Organopolysiloxanen, die ungesättigt vorliegen, ausgewählt sind;
(d) Homopolymeren und Copolymeren von Vinylalkohol;
(e) Homopolymeren von Vinylestern oder Vinylamiden und/ oder Allylestern oder Allylamiden und/ oder Methallylestern oder Methallylamiden und deren Copolymeren mit Comonomeren, die unter ungesättigten Carbonsäuren, Sulfonsäuren, Phosphonsäuren, Vinylestern und Vinylethern, Olefinen, Styrol, substituierten Styrolen, Fluor- und Perfluorolefinen, Perfluoralkyl(meth)acrylaten, fluorierten Vinylverbindungen und Organosilarien, Organosiloxanen oder Organopolysiloxanen, die ungesättigt vorliegen, ausgewählt sind;
(f) Polyethern;
(g) Polyestern;
(h) Homo- und Copolymeren von Olefinen oder Cycloolefinen;
(i) Polyamiden und Polyesteramiden;
(j) Polyurethanen und Polyharnstoffen, die Polyethersequenzen, Polyestersequenzen und/oder Polyorganosiloxansequenzen enthalten können;
(k) fluorierten Polymeren;
(l) natürlichen Polymeren und natürlichen Polymeren, die modifiziert sind (künstlichen Polymeren);
(m) Polyorganosiloxanen;
(n) Polyorganophosphazenen;
(o) Polysilanen -(SiR^{a}R^{b}-)ₙ, Polycarbosilanen -(SiR^{a}R^{b}-CR^{c}R^{d}-)ₙ und Polysilazanen -(-SiR^{a}R^{b}-NR^{c}-)ₙ und
(p) Gemischen dieser Polymere,
oder einem ihrer Vorläufermaterialien,
das nach dem Verdampfen der flüchtigen Fraktion eine vernetzte Struktur bildet.

2. Verfahren nach Anspruch 1, **dadurch gekennzeichnet, dass** das Gemisch, das das organisch-anorganische Hybridmaterial ergibt, ferner ein Lösungsmittel enthält (Komponente C).

3. Verfahren nach einem der vorhergehenden Ansprüche, **dadurch gekennzeichnet, dass** die Verbindung (A) eine metallische Verbindung ist, die ausgewählt ist unter:
(i) Oxiden von Übergangsmetallen der Gruppen IB bis VIIB oder aus der Gruppe der Lanthanoide des Periodensystems der Elemente,
(ii) Oxiden von Aluminium, Bor, Silicium oder Zinn, und
(iii) Aluminiumphosphaten.

4. Verfahren nach Anspruch 3, **dadurch gekennzeichnet, dass** die Verbindung (A) unter Titanoxid, Eisenoxid und Zirconiumoxid ausgewählt ist.

5. Verfahren nach Anspruch 1 oder 2, **dadurch gekennzeichnet, dass** die Verbindung (A) eine Metall-organische Verbindung ist, die ausgewählt ist unter:
(1) Verbindungen, die einer der folgenden Formeln entsprechen:
(Ia) M(OR₁)ₙ
(Ib) R-M-(OR₁)ₙ₋₁
(Ic) (OR₁)ₙ₋₁-M-R"-M(OR₁)ₙ₋₁ 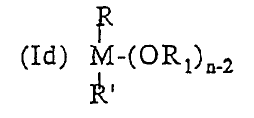
worin M ein Übergangsmetall der Gruppen IB bis VIIB des Periodensystems der Elemente oder ein Aluminiumatom, Boratom, Siliciumatom oder Zinnatom und vorzugsweise Si, Ti oder Zr bedeutet,
n der Valenz von M entspricht,
R₁ eine geradkettige oder verzweigte C₁₋₃₀-Alkylgruppe und vorzugsweise C₁₋₆-Alkylgruppe ist,
R und R' unabhängig voneinander eine geradkettige oder verzweigte Alkylgruppe, eine Cycloalkylgruppe oder eine Arylgruppe, die gegebenenfalls substituiert ist, bedeuten, wobei die Gruppen R und R' selbst befähigt sein können, mit dem organischen oder siliconierten Polymer (B) zu reagieren, oder einen Substituenten tragen können, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
R" eine geradkettige oder verzweigte Alkylengruppe, Cycloalkylengruppe oder Arylengruppe, die gegebenenfalls substituiert ist, bedeutet, wobei R" selbst mit dem organischen oder siliconierten Polymer (B) reagieren kann oder einen Substituenten aufweisen kann, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
(2) Koordinationskomplexen der oben angegebenen Verbindungen der Formel (Ia) bis (Id), die einer der folgenden Formeln entsprechen:
(IIa) M(OR₁)ₙ₋ₓ (X)ₓ
(IIb) R-M(OR₁)ₙ₋₁₋ₓ (X)ₓ
(IIc) (X)ₓ (OR₁)ₙ₋₁₋ₓM-R"-M-(O-R₁)-ₙ₋₁₋ₓ (X)ₓ 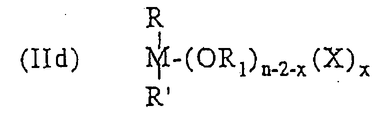
wobei M ein Übergangsmetall der Gruppen IB bis VIIB des Periodensystems der Elemente oder ein Aluminiumatom, Boratom, Siliciumatom oder Zinnatom und vorzugsweise Ti oder Zr ist,
X einen einfach koordinierten oder einzähnigen Liganden bedeutet, der ein Stickstoffatom, Phosphoratom, Schwefelatom oder Sauerstoffatom enthält und einen Substituenten, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist, und eine Gruppe enthalten kann, die unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
x die Anzahl der Liganden X bedeutet, und
n, R, R₁, -R' und R" die für die Formeln (Ia) bis (Id) angegebenen Bedeutungen aufweisen, und
(3) Chelatkomplexen der folgenden Formel:
(III) M(OR₁)n-t,X (X')ₓ
worin bedeuten
M ein Übergangsmetall der Gruppen IB bis VIIB des Periodensystems der Elemente oder ein Aluminiumatom, Boratom, Siliciumatom oder Zinnatom und vorzugsweise Ti oder Zr,
X'- eine zur Chelat-Bildung befähigte Gruppe oder einen mehrzähnigen Liganden, der ein Stickstoffatom, Phosphoratom, Schwefelatom und/oder ein Sauerstoffatom enthalten und einen Substituenten aufweisen kann, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat
oder von EDTA abgeleiteten Gruppen und deren Derivaten ausgewählt ist, und eine Gruppe enthalten kann, die unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
b der Koordinationsvalenz des Liganden X' entspricht und mindestens 2 beträgt,
x gleich der Anzahl der Liganden X' ist, und
R₁ und n die oben für die Formeln (Ia) bis (Id) angegebenen Bedeutungen aufweisen.

6. Verfahren nach Anspruch 5, **dadurch gekennzeichnet, dass** der einfach koordinierte Ligand X unter den Carbonsäuren und Ketonen ausgewählt ist.

7. Verfahren nach Anspruch 5, **dadurch gekennzeichnet, dass** der mehrzähnige Ligand X' unter den β-Diketonen, β-Ketoestem, β-Ketoaminen, α- und β-Hydroxysäuren, gegebenenfalls β-hydroxylierten Aminosäuren, Salicylsäure und deren Derivaten ausgewählt ist.

8. Verfahren nach Anspruch 7, **dadurch gekennzeichnet, dass** der mehrzähnige Ligand X' unter Acetoxyethylmethacrylat, Methyl-α-hydroxymethacrylat, ε-N-Methacryloyl-L-lysin, 4-Methacrylaminosalicylsäure und 5-Methacrylaminosalicylsäure ausgewählt ist.

9. Verfahren nach einem der Ansprüche 1 bis 2 und 5 bis 8, **dadurch gekennzeichnet, dass** die Metall-organische Verbindung unter Tetraethoxysilan, Tetra-*n*-propylorthotitanat, Tetra-*iso*propylorthotitanat, Tetra-*n*-propylzirconat, Tetra-*iso*-propylzirconat, Methyltriethoxysilan, Tetraethoxytitan, Tetrabutoxytitan, Triethoxyeisen und Triethoxywolfram ausgewählt ist.

10. Verfahren nach Anspruch 9, **dadurch gekennzeichnet, dass** es sich bei der Metall-organischen Verbindung um Tetra-iso-propylorthotitanat, Tetra-*n*-propylorthotitanat, Tetra-*iso*-propylzirconat und Tetra-*n*-propylzirconat handelt.

11. Verfahren nach einem der Ansprüche 1 bis 10, **dadurch gekennzeichnet, dass** die metallische oder Metall-organische Verbindung (A) in einer Menge von 1 bis 99 Gew.-%, vorzugsweise 5 bis 80 Gew.-% und insbesondere 10 bis 70 Gew.-%, bezogen auf das Gewicht des organisch-anorganischen Hybridmaterials enthalten ist.

12. Verfahren zur Verbesserung der Abriebfestigkeit eines Make-ups oder eines Lippenstifts, **dadurch gekennzeichnet, dass** auf die Haut oder die Lippen eine Make-up-Zusammensetzung oder ein Lippenstift aufgetragen wird, in die ein organisch-anorganisches Hybridmaterial eingebracht wurde, das durch ein Sol-Gel-Verfahren aus einem Gemisch erhalten wurde, das enthält:
(A) mindestens eine metallische oder Metall-organische Verbindung und
(B) mindestens ein organisches oder siliconiertes Polymer, das unter einem statistischen, sequentiellen und/ oder gepfropften Homopolymer oder Copolymer ausgewählt ist, welches ausgewählt ist unter:
(a) Alkyloxazolin-Homopolymeren und Alkyloxazolin-Copolymeren;
(b) Homopolymeren und Copolymeren von (Meth)acrylsäure, Crotonsäure, Maleinsäure, Itaconsäure, Styrol-sulfonsäure, Acrylamido-2-methylpropan-sulfonsäure, 2-Sulfoethylmethacrylat, Vinylsulfonsäure und/oder Vinylphosphonsäure;
(c) Homopolymeren von Acrylestern, Methacrylestern, Acrylamiden oder Methacrylamiden und deren Copolymeren mit Comonomeren, die unter ungesättigten Carbonsäuren, Sulfonsäuren, Phosphonsäuren, Vinylestern und Vinylethern, Olefinen, Styrol, substituierten Styrolen, Fluor- oder Perfluorolefinen, Perfluoralkyl(meth)acrylaten, fluorierten Vinylverbindungen und Organosilanen, Organosiloxanen oder Organopolysiloxanen, die ungesättigt vorliegen, ausgewählt sind;
(d) Homopolymeren und Copolymeren von Vinylalkohol;
(e) Homopolymeren von Vinylestern oder Vinylamiden und/oder Allylestern oder Allylamiden und/oder Methallylestern oder Methallylamiden und deren Copolymeren mit Comonomeren, die unter ungesättigten Carbonsäuren, Sulfonsäuren, Phosphonsäuren, Vinylestern und Vinylethern, Olefinen, Styrol, substituierten Styrolen, Fluor- und Perfluorolefinen, Perfluoralkyl(meth)acrylaten, fluorierten Vinylverbindungen und Organosilanen, Organosiloxanen oder Organopolysiloxanen, die ungesättigt vorliegen, ausgewählt sind;
(f) Polyethern;
(g) Polyestern;
(h) Homo- und Copolymeren von Olefinen oder Cycloolefinen;
(i) Polyamiden und Polyesteramiden;
(j) Polyurethanen und Polyharnstoffen, die Polyethersequenzen, Polyestersequenzen und/oder Polyorganosiloxansequenzen enthalten können;
(k) fluorierten Polymeren;
(l) natürlichen Polymeren und natürlichen Polymeren, die modifiziert sind (künstlichen Polymeren);
(m) Polyorganosiloxanen;
(n) Polyorganophosphazenen;
(o) Polysilanen -(SiR^{a}R^{b}-)ₙ, Polycarbosilanen -(SiR^{a}R^{b}-CR^{c}R^{d}-)ₙ und Polysilazanen -(-SiR^{a}R^{b}-NR^{c}-)ₙ und
(p) Gemischen dieser Polymere,
oder einem ihrer Vorläufermaterialien,
das nach dem Verdampfen der flüchtigen Fraktion eine vernetzte Struktur bildet.

13. Verfahren nach Anspruch 12, **dadurch gekennzeichnet, dass** das Gemisch, das das organisch-anorganische Hybridmaterial ergibt, ferner mindestens ein Lösungsmittel (Komponente (C)) enthält.

14. Verfahren nach Anspruch 12 oder 13, **dadurch gekennzeichnet, dass** das organisch-anorganische Hybridmaterial homogen in die kosmetische Zusammensetzung eingearbeitet wird.

15. Verfahren nach einem der Ansprüche 12 bis 14, **dadurch gekennzeichnet, dass** die Verbindung (A) eine metallische Verbindung ist, die ausgewählt ist unter:
(i) Oxiden von Übergangsmetallen der Gruppen IB bis VIIB oder aus der Gruppe der Lanthanoide des Periodensystems der Elemente,
(ii) Oxiden von Aluminium, Bor, Silicium oder Zinn, und
(iii) Aluminiumphosphaten.

16. Verfahren nach Anspruch 15, **dadurch gekennzeichnet, dass** die Verbindung (A) unter Titanoxid, Eisenoxid und Zirconiumoxid ausgewählt ist.

17. Verfahren nach einem der Ansprüche 12. bis 14, **dadurch gekennzeichnet, dass** die Verbindung (A) eine Metall-organische Verbindung ist, die ausgewählt ist unter:
(1) Verbindungen, die einer der folgenden Formeln entsprechen:
(Ia) M(OR₁)ₙ
(Ib) R-M-(OR₁)ₙ₋₁
(Ic) (OR₁)ₙ₋₁-M-R"-M(OR₁)ₙ₋₁ 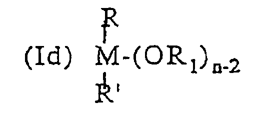
worin M ein Übergangsmetall der Gruppen IB bis VIIB des Periodensystems der Elemente oder ein Aluminiumatom, Boratom, Siliciumatom oder Zinnatom und vorzugsweise Si, Ti
oder Zr bedeutet,
n der Valenz von M entspricht,
R₁ eine geradkettige oder verzweigte C₁₋₃₀-Alkylgruppe und vorzugsweise C₁₋₆-Alkylgruppe ist,
R und R' unabhängig voneinander eine geradkettige oder verzweigte Alkylgruppe, eine Cycloalkylgruppe oder eine Arylgruppe, die gegebenenfalls substituiert ist, bedeuten, wobei die Gruppen R und R' befähigt sein können, selbst mit dem organischen oder siliconierten Polymer (B) zu reagieren, oder einen Substituenten tragen können, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
R" eine geradkettige oder verzweigte Alkylengruppe, Cycloalkylengruppe oder Arylengruppe, die gegebenenfalls substituiert ist, bedeutet, wobei R" selbst mit dem organischen oder siliconierten Polymer (B) reagieren kann oder einen Substituenten aufweisen kann, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
(2) Koordinationskomplexen der oben angegebenen Verbindungen der Formel (Ia) bis (Id), die einer der folgenden Formeln entsprechen:
(IIa) M(OR₁)ₙ₋ₓ (X)ₓ
(IIb) R-M(OR₁)ₙ₋₁₋ₓ (X)ₓ
(IIc) (X)ₓ (OR₁)ₙ₋₁₋ₓM-R"-M-(O-R₁)ₙ₁₋ₓ (X)ₓ 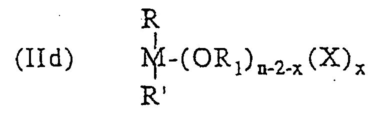
wobei M ein Übergangsmetall der Gruppen IB bis VIIB des Periodensystems der Elemente oder ein Aluminiumatom, Boratom, Siliciumatom oder Zinnatom und vorzugsweise Ti oder Zr ist,
X einen einfach koordinierten oder einzähnigen Liganden bedeutet, der ein Stickstoffatom, Phosphoratom, Schwefelatom oder Sauerstoffatom enthält und einen Substituenten aufweisen kann, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist, und eine Gruppe enthalten kann, die unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
x die Anzahl der Liganden X bedeutet, und
n, R, R₁, R' und R" die für die Formeln (Ia) bis (Id) angegebenen Bedeutungen aufweisen, und
(3) Chelatkomplexen der folgenden Formel:
(III) m(OR₁)_{n-bx} (X')ₓ
worin bedeuten:
M ein Übergangsmetall der Gruppen IB bis VIIB des Periodensystems der Elemente oder ein Aluminiumatom, Boratom, Siliciumatom oder Zinnatom und vorzugsweise Ti oder Zr,
X' eine zur Chelat-Bildung befähigte Gruppe oder einen mehrzähnigen Liganden, der ein Stickstoffatom, Phosphoratom, Schwefelatom und/oder ein Sauerstoffatom enthalten und einen Substituenten aufweisen kann, der unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist, und eine Gruppe enthalten kann, die unter den ethylenisch ungesättigten Gruppen, halogenierten Gruppen, hydroxylierten Gruppen, carboxylierten Gruppen, Thiolen, Epoxy, Estern, Amino, Amido, Aminosäuren, Polypeptiden, Urethan, Ureido, Acetoacetat, Ethylacetoacetat oder von EDTA abgeleiteten Gruppen und deren Derivaten ausgewählt ist,
b der Koordinationsvalenz des Liganden X' entspricht und mindestens 2 beträgt,
x gleich der Anzahl der Liganden X' ist, und
R₁ und n die oben für die Formeln (Ia) bis (Id) angegebenen Bedeutungen aufweisen.

18. Verfahren nach Anspruch 17, **dadurch gekennzeichnet, dass** der einfach koordinierte Ligand X unter den Carbonsäuren und Ketonen ausgewählt ist.

19. Verfahren nach Anspruch 17, **dadurch gekennzeichnet, dass** der mehrzähnige Ligand X' unter den β-Diketonen, β-Ketoestern, β-Ketoaminen, α- und β-Hydroxysäuren, gegebenenfalls β-hydroxylierten Aminosäuren, Salicylsäure und deren Derivaten ausgewählt ist.

20. Verfahren nach Anspruch 19, **dadurch gekennzeichnet, dass** der mehrzähnige Ligand X' unter Acetoxyethylmethacrylat, Methyl-α-hydroxymethacrylat, ε-N-Methacryloyl-L-lysin, 4-Methacrylaminosalicylsäure und 5-Methacrylaminosalicylsäure ausgewählt ist.

21. Verfahren nach einem der Ansprüche 12 bis 14 und 17 bis 20, **dadurch gekennzeichnet, dass** die Metall-organische Verbindung unter Tetraethoxysilan, Tetra-*n*-propylorthotitanat, Tetra-*iso*propylorthotitanat, Tetra-*n*-propylzirconat, Tetra-*iso*-propylzirconat, Methyltriethoxysilan, Tetraethoxytitan, Tetrabutoxytitan, Triethoxyeisen und Triethoxywolfram ausgewählt ist.

22. Verfahren nach Anspruch 21, **dadurch gekennzeichnet, dass** es sich bei der Metall-organischen Verbindung um Tetra-*iso*-propylorthotitanat, Tetra-*n*-propylorthotitanat, Tetra-*iso*-propylzirconat und Tetra-*n*-propylzirconat handelt.

23. Verfahren nach einem der Ansprüche 12 bis 22, **dadurch gekennzeichnet, dass** die metallische oder Metall-organische Verbindung (A) in einer Menge von 1 bis 99 Gew.-%, vorzugsweise 5 bis 80 Gew.-% und insbesondere 10 bis 70 Gew.-%, bezogen auf das Gewicht des organisch-anorganischen Hybridmaterials enthalten ist.

24. Verfahren nach einem der Ansprüche 12 bis 23, **dadurch gekennzeichnet, dass** das organisch-anorganische Hybridmaterial in einer Menge von 10 bis 90 Gew.-% und vorzugsweise 15 bis 70 Gew.-%, bezogen auf das Gesamtgewicht der fertigen kosmetischen Zusammensetzung, eingearbeitet wird.

25. Verfahren nach einem der vorhergehenden Ansprüche, **dadurch gekennzeichnet, dass** es sich bei den Polyorganosiloxanen um Polydimethylsiloxane, hydroxylierte Polydimethylsiloxane, Polydimethylsiloxane, die Ethylenoxid- und/oder Propylenoxid-Propfzweige enthalten, oder sequentielle Polymere EO/PO-PDMS-EO / PO handelt.

26. Verfahren nach Anspruch 25, **dadurch gekennzeichnet, dass** das organische oder siliconierte Polymer (B) ein hydroxyliertes Polydimethylsiloxan ist.

27. Verfahren nach einem der Ansprüche 2 bis 26, **dadurch gekennzeichnet, dass** das Lösungsmittel unter den Alkoholen, insbesondere geradkettigen oder verzweigten, aliphatischen C₁₋₆-Alkoholen, flüchtigen oder nicht flüchtigen Siliconölen, flüchtigen oder nicht flüchtigen Kohlenwasserstoffölen, pflanzlichen Ölen, Mineralölen, synthetischen Ölen oder tierischen Ölen oder deren Gemischen ausgewählt ist.

28. Verfahren nach einem der vorhergehenden Ansprüche, **dadurch gekennzeichnet, dass** die Polymerkomponente (B) im Allgemeinen in einer Menge von 1 bis 99 Gew.-%, vorzugsweise 5 bis 80 Gew.-% und insbesondere 10 bis 70 Gew.-%, bezogen auf das Gewicht des organisch-anorganischen Hybridmaterials, enthalten ist.

29. Verfahren nach einem der vorhergehenden Ansprüche, **dadurch gekennzeichnet, dass** die kosmetische Zusammensetzung eine Zusammensetzung zur Pflege oder zum Schminken ist, die auf die Haut oder die Lippen aufgetragen werden kann.

30. Verfahren nach Anspruch 29, **dadurch gekennzeichnet, dass** es sich bei der Zusammensetzung zum Schminken um Make-up oder Lippenstift handelt.
